# EUROPEAN PATENT SPECIFICATION

(11) **EP 1 383 375 B1**
(45) Date of publication and mention of the grant of the patent: **23.08.2006**
(21) Application number: 02725786.4
(22) Date of filing: 23.04.2002
(51) Int. Cl.: A01N 25/00, A61K 47/00, A61K 47/30, A61K 47/32, A61K 47/34, A61K 9/48

(54) **A RELEASE PHARMACEUTICAL CONSTRUCT FOR GASTRIC RETENTION**
PHARMAZEUTISCHES FREIGABEKONSTRUKT ZUR MAGENRETENTION
STRUCTURE PHARMACEUTIQUE D'APPORT SE MAINTENANT DANS L'ESTOMAC

(30) Priority: 23.04.2001 US 285717 P; 19.04.2002 US 126828
(43) Date of publication of application: 28.01.2004
(73) Proprietor: Kos Life Sciences, Inc., Weston, FL 33326 (US)
(72) Inventor: GUTIERREZ-ROCCA, Jose, Miami, FL 33172 (US); DUNNE, Josephine, Plantation, FL 33324 (US); LALLY, Matthew, Fort Lauderdale, FL 33324 (US)
(74) Representative: Forstmeyer, Dietmar
(86) International application number: PCT/US2002/012858
(87) International publication number: WO 2002/085112

(56) References cited:
- US-A- 4 085 168
- US-A- 4 851 232

## Description

### BACKGROUND OF THE INVENTION

### Field of the Invention

This invention relates to a pharmaceutical construct, and, more particularly, to a construct comprising (a) a selected medicament and at least, (b) a first layer comprising a superporous hydrogel containing a first functional group, having a surface to which said medicament is associated, to form at least one associated surface.

### Description of the Related Art

There are many drugs whose preferred sites of absorption are the duodenum, jejunum and ileum- located soon after the stomach of a mammal, such as a human being. They may also be absorbed subsequently in the gastrointestinal tract, but in many cases, less so. Accordingly, these areas can be treated as the primary target area for absorption of such drugs.

In order to reduce the number of doses of a drug required over a time, it is useful to increase the amount of time that a platform responsible for drug delivery remains in a suitable position to deliver drug for absorption in these areas. Since the body moves food through the gastro-intestinal ("GI") tract steadily in one direction, the delivery platform must be positioned on or before these areas of maximum absorption. An ideal place to locate the drug delivery platform is the stomach, which comes immediately before these target areas.

In order to have a platform deliver a drug from the stomach, it must do the following:
(1) remain intact for the duration of the drug delivery period, despite the pressures and the pH in the stomach;
(2) remain in the stomach for that same duration, despite efforts from the stomach muscles to remove it;
(3) release the drug in suitable form and with a suitable rate; and
(4) be non-toxic. In this regard, the stomach has been observed to exert pressures equivalent to 100-130cmH₂O. These pressures are a result of the peristaltic waves produced by the stomach muscles, which are used to convert food into chyme by mixing it with gastric fluid, which contains hydrochloric acid and digestive enzymes. Housekeeper waves are a specific type of peristaltic wave and are used to break up indigestible food. It is these waves that will present the most danger to the delivery platform. The platform used must be able to resist pressures produced by such waves, as well as higher pressures which can result from localized shear forces.

The chyme and other remaining food is released from the stomach periodically through the pyloric sphincter at the bottom of the stomach. The pyloric sphincter can dilate to allow particles of undigested food and chyme out of the stomach into the GI tract. It is generally thought that particles greater than 1 to 2mm are retained in the stomach, although there is no consensus on this point.

The physiological behavior of the stomach is usually determined by whether it contains food or is empty. Food is mixed and partially digested in the distal stomach (antrum). As the stomach undergoes contractions, partially digested material is discharged into the small intestine and non-digested material is retropelled into the main part of the stomach for further digestion. In the fed state, non-digestible material is not generally able to leave the stomach. At the end of a digestive period, the stomach enters the fasting stage and begins a cycle called the interdigestive myolectric motor cycle or IMMC.

The IMMC can be considered to be divided into four phases: (1) phase 1 is an approximately one hour period with no contractions; (2) phase 2 is about a forty minute period of intermittent potentials and contractions that increase in intensity over time; (3) phase 3 is a relatively short period, generally between about five to fifteen minutes, of intense contractions (the "housekeeper wave") that completely empties the stomach; and (4) phase 4 is a short transitory period between the intense activity of phase 3 and the quiescence of phase 1. The different phases move distally from the stomach to the terminal ileum over an approximately two hour period as the cycle is repeated. Since the cycle is interrupted by the receipt of food by the stomach, it is possible to delay the emptying phase. phase 3, by maintaining a fed state. However, it is not practical to regularly maintain the fed state over a long period of time. Consequently, a need exists for a delivery device or platform that can remain in the stomach for a significant period, whether in the fed or fasted state, and deliver active agent to the stomach over a prolonged period of time.

A variety of studies have been conducted in dog and in man to determine sizes of objects that would be retained in the stomach during the fed stage and also in the fasting stage when IMMC is present. Khosla and Davis, *International Journal of Pharmaceuticals*, Vol. 62 (1990), pages R9-R11, have reported that a particle size less than 2 mm generally results in emptying from the stomach of the dog. Non-disintegrating tablets having sizes of 7, 11 and 13 mm in diameter were emptied from the human stomach, but the larger sized tablets tended to remain in the stomach longer than the small sized tablets. Tablets larger than 11 mm tended to be emptied only during the IMMC. Davis et al., *Pharmaceutical Research,* vol. 8, No. 10 (1991) has described retention of radio-telemetry capsules having a size of 25x8 mm in the stomach of human subjects past phase 3 of the IMMC. Timmermans et al. *Journal of Pharmaceutical Sciences.* vol. 82, No. 8 (1993) has reported the mean resting pyloric diameter in humans is 12.8±7.0 mm. Accordingly, it is important that gastric retentive delivery vehicles are adapted to disintegrate, dissolve or erode to sizes that permit eventual elimination of the vehicle without causing gastric obstruction.

Various attempts to provide active agent delivery devices that remain in the stomach for extended periods or time have been described previously. For example, U.S. Pat. No. 4,851,232 describes a hydrogel reservoir containing tiny pills having an active agent core surrounded by a wall controlling delivery of active agent to the stomach. The hydrogel swells in the stomach to facilitate retention of the active agent reservoir in the stomach over time.

U.S. Pat. No. 4.871,548 describes a dosage form including a mixture of low and high number average molecular weight hydroxypropylmethylcellulose polymers and active agent that swells when in the stomach.

U.S. Pat. No. 4,767,627 describes substantially planar devices formed of bioerodible polymer including active agent that may be compressed and folded for oral administration and then released and unfolded in the stomach, where the devices are to be retained over an extended period of time. The devices have a longest diameter of between 1.6 and 5 cm. It is suggested that as an alternative to incorporating the active agent into the device a controlled release active agent module, mechanically or osmotically driven can be glued or tethered to the device.

U.S. Pat. No. 5,443,843 describes a plurality of compressible retention arms and an attached controlled release device which in the expanded form resists gastrointestinal transit. The system can have a collar or a belt for receiving and holding an active agent-containing, orally-administrable controlled release device. In a fully expanded configuration for human use, the system is described as having minimum and maximum dimensions of 2.5 and 6.0 centimeters, respectively.

U.S. Pat. No. 5,007,790 describes a sustained release active agent dosage form in the form of a capsule or tablet that includes a plurality of hydrophilic water-swellable, cross-linked polymer particles that swell in the stomach to promote gastric retention and permit gastric fluid to penetrate the particles to dissolve active agent and deliver it to the stomach in the solution state. The particles are indicated to retain their physical integrity over the dosing period. Initially sized particles, indicated to be preferably spherical, are disclosed to be in the range of 50 µm to 2 mm, swell to a size of about 3 mm. A plurality of particles are packed into a capsule for administration to a patient.

U.S. Pat. No. 5,582,837 describes a dosage form similar to that of U.S. Pat. No. 5,007,790, without the use of a cross-linked hydrophilic polymer. The particles are described as slippery and soft, preferably spherical, and having dimensions on the order of 6 to 18 mm in the swollen state. The particles can be packed into capsules containing 7-25 spherical particles, depending on the size, or formulated into tablets that contain from 2-25 spherical particles.

The use of albumin-cross-linked polyvinylpyrolidone hydrogels to deliver flavin mononucleotide to dogs has been described by Park et al. In *Journal of Controlled Release,* Vol. 19 (1992) pages 131-134. The hydrogels were maintained in the stomachs of dogs for extended periods, even in the fasted state. Gels with a glassy core tended to remain in the stomach longer than hydrogels without the glassy core. Control of the size of the core was attempted by administration of water in the stomach.

While it is important that the delivery device or platform be adapted to remain in the stomach for a prolonged period, it is also important that the device deliver active agent in a controlled manner. Delivery systems, such as those described below, are representative of the many different systems have been suggested for such controlled delivery of active agents over a prolonged period of time.

For example, U.S. Pat. No. 4,290,426 to Lusted et al describes a cylindrical dispenser for releasing a beneficial agent into a fluid environment at a rate that is governed by the fluid induced relaxation of a polymeric agent contained within the dispenser. The cylindrical dispenser includes an impermeable container that has within it a reservoir and a passageway from the reservoir to the exterior of the container. The reservoir contains a polymer and a beneficial agent. The polymer imbibes fluid from the environment and thereby undergoes relaxation, releasing the beneficial agent from the device. The amount of agent released is dependent on the rate of relaxation of the polymer over time.

Coated dosage forms have also been suggested for delivery of a controlled amount of a beneficial agent over a prolonged period of time. U.S. Pat. No. 5,256,440 describes a process for producing a film coated dosage form. A continuous groove is inscribed in a dosage form core. A latex film is coated onto the core, the groove defining a fixed zone and a detachable zone for the film. The detachable portion of the latex film detaches when it is exposed to the environment of use, thereby exposing a discrete portion of the dosage form core surface. The remainder of the film remains attached to the dosage form core. The exposed portion of the dosage form surface erodes and releases active agent to the environment of use.

Coated tablets for constant and prolonged active agent release are described by Conte et al. in *J. Controlled Release,* Vol. 26, (1993) pages 39-47. These GEOMATRIX^{™} Systems are swellable matrices that are coated or tableted with polymeric barrier layers. Release performances of the systems are modulated as a result of the reduction of the releasing surface exposed to the dissolution medium by the polymeric barrier layer coatings. As the extent of coating of the system's surface is increased, the release kinetics of the system shift toward constant release. These systems are further described in U.S. Pat. No. 4,839,177 to Colombo et al.

U.S. Pat. No. 5,534,263, describes a dosage form useful for the prolonged delivery of an active agent formulation in the form of a matrix having two or more insoluble bands on the surface of the matrix. The exposed surfaces of the matrix erode in a manner that creates additional surface areas to provide for prolonged release of an active agent formulation with determined release profiles.

Additional oral, controlled-release dosage forms include elementary osmotic pumps, such as those described in US. Pat, No. 3,845,770, mini-osmotic pumps such as those described in U.S. Pat. Nos. 3,995,631, 4,034,756 and 4,111, 202, and multi-chamber osmotic systems referred to as push-pull, push-melt and push-stick osmotic pumps, such as those described in U.S. Pat. Nos. 4,320,759, 4,327,725, 4,449,983, 4,765,989 and 4,940,465.

Administration of acyclovir by sipped solution over a four-hour period has been described in *Br. J. clin. Pharmac*., 21, 459-462 (1986) to achieve an increased contact time with the human stomach and the gastrointestinal tract. The total amount of acyclovir absorbed was increased over that observed with administration of acyclovir tablets. The influence of food on gastric retention time and the absorption of acyclovir tablets has been reported in *International Journal of Pharmaceutics*, Vol. 38 (1987), pages 221-225. As reported there, compared to a lighter meal, the heavier meal slowed the rate of gastric emptying, prolonged small intestinal transit time and decreased absorption of the active agent.

### SUMMARY OF THE INVENTION

This invention relates to a pharmaceutical construct, and, more particularly, to a construct comprising a selected medicament associated with at least one surface of a first layer comprising a first superporous hydrogel, where at least a portion of the at least one surface is covered by a second layer comprising a second superporous hydrogel which is attracted or linked to the first superporous hydrogel by means of functional groups on each respective layer.

### BRIEF DESCRIPTION OF THE DRAWINGS

The present invention will be more readily understood by reference to the following drawing taken in conjuction with the detailed description, wherein:
FIG. 1 is a partial cross-sectional view of a pharmaceutical construct or platform of the invention;
FIG. 2 is a schematic view of an alternative construct or active agent dosage form of the invention; and
FIGS. 3, 4 and 5 are schematic views of other constructs of the invention.

### DETAILED DESCRIPTION OF THE INVENTION

A superporous hydrogel ("SPH") is a hydrogel that has super size pores in its interior. The pores are in excess of 100 µm, usually several hundred micrometers up to the millimeter range. The SPH can swell in a compatible liquid environment to an equilibrium size in less than a minute. The intended environment is the stomach of an animal, e.g. a human being, such as the gastric fluids contained therein.

The pores of the SPH are connected so that they can act as channels through which water and other fluids can enter. By comparison, regular hydrogels have pores equivalent in size to the spaces between the polymer chains that make them up. Absorption of water into such systems is only by diffusion. Due to the highly porous nature of superporous hydrogels, the dry material may swell to hundreds of times its original size when placed in water or in the stomach of an animal, e.g. a human being. The swollen gels may also be made to have high mechanical strength. Changing the composition can alter the actual properties of the material. Composites can be made with Ac-Di-Sol brand croscarmellose sodium for strength, and platicizers can be used to make the material more flexible.

It is to be noted that the SPH can be made biodegradable, e.g. degradable by pepsin in the stomach of a human being or other animal. For example, the SPH can be made up in capsule form and upon ingestion by the patient, e.g. human being, it swells to a size of several centimeters which is too large to be emptied by the stomach of such patient. A drug associated therewith is released at a controlled rate and the SPH degrades, e.g. by the action of the pepsin, and ultimately empties from the stomach.

As used herein the term "associate", "associated" or "association" is meant that a selected medicament is combined with or mixed with at least one SPH or is encapsulated or entrapped by the resultant polymeric matrix or forms part of the resultant polymeric matrix or is deposited on a surface of such matrix of at least one SPH.

SPH's are well known in the art as are their methods of synthesis. In this regard, reference is made to Chen et al., "SYNTHESIS OF SUPERPOROUS HYDROGELS," pp. 53-62 44; Chen et al., *Journal of Biomedical Material Research,* 1 (1999); U.S. Patent No. 5,750,585; Gemeinhart et al., *Proceed. Int'l Symp. Control. Rel. Bioact. Mater.* 26 (1999), 5449, all of which are incorporated hereinto by reference in their entirety.

Suitable monomers which are polymerized to make a SPH are acrylic acid, acrylamide; vinylpyrrolidone; sulfopropyl acrylate, hydroxyethyl methacrylate; hydroxypropyl methacrylate and hydroxyethyl acrylate. Preferred SPHs are P(AA-SPAK), P(AA-AM), P(AA-HEMA-AM), P(AA), P(AA-HEMA-HGA),. Most preferred SPHs are P(AA-SPAK), P(AA-AM), P(AA-HEMA-AM), P(AA), P(AA-HEMA-HGA), where the individual abbreviations are explained in TABLE I below and where "P" means poly.

The SPH monomer can be combined with a suitable cross-linker to link the SPH polymer chains which are formed. Some typical cross-linkers include, N'-methylene-bis-acrylamide, polyethylene glycol diacrylate, diethylene glycol diacrylate, divinyl glycol, etc. The SPH monomer and the cross linker or cross linker mixtures are combined in appropriate amounts, e.g. 50 ml of AA to 0.5 g of BN acrylamide, and then mixed with water under proper pH control e.g. 3 to 8, and subjected to foaming agents, initiators to form capillary channels.

**TABLE I**

| **Ingredient** | **Acronym** |
|---|---|
| Acrylic Acid | AA |
| Acrylamide | AM |
| Vinylpyrrolidone | VP |
| Hydroxyethyl methacrylate | HEMA |
| 3-sulfopropyl acrylate, potassium salt | SPAK |
| N,N'-methylenebisacrylamide | Bis |
| Ammonium persulfate | APS |
| 1Hydroxypropyl methacrylate | HPMA |
| N,N,N',N'-tetramethylenediamine | TEMED |
| Pluronic F127 | PF127 |
| Hydroxyethyl acrylate | HEA |
| Diethylene glycol diacrylate | DEGDA |
| Polyethylene glycol diacrylate | PEGDA |
| Ammonium Persulfate | APS |

Other polymerization additives may also be employed, such as a foaming agent, e.g. Pluronic F127, a surfactant, a free radical polymerization enhancing agent e.g. tetramethylenediamine, ammonium persulfate, a strength/flexibility enhancing agent, e.g. Ac-Di-Sol® (croscarmellose sodium), and plasticizers, e.g. glycerol.

Typically, the time elapse between initiation of polymerization and foaming relates to the pore size of the resultant SPH. However, the pH must be carefully controlled because it affects the rate of foaming. The rate of polymerization is greater under basic conditions.

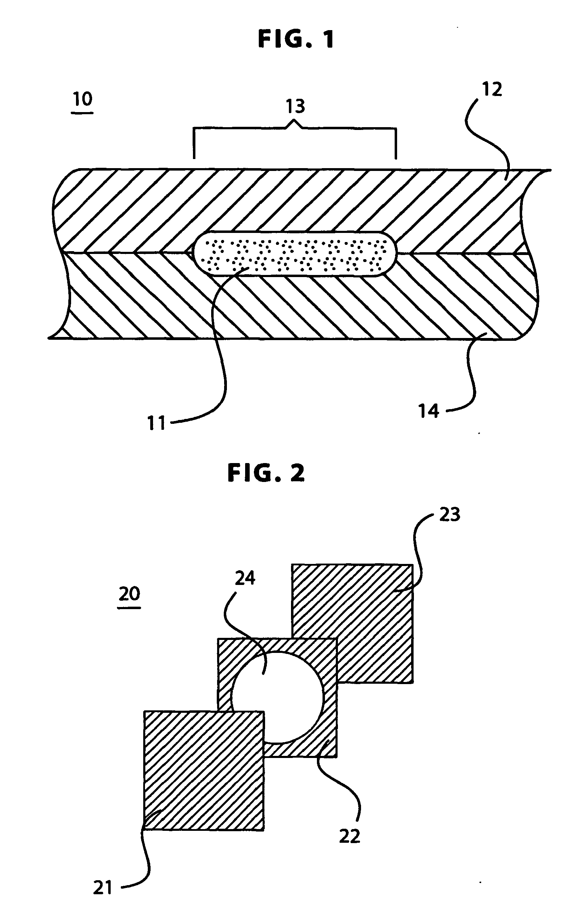

Referring to FIG. 1, there is shown a partial cross-sectional view of a first construct of the subject invention. A suitable medicament active ingredient is selected. The medicament ingredient is typically contained in a controlled release formulation 11. Typically a particulate medicament is enclosed or coated with an outer time or controlled release layer, e.g. a shellac layer, cellulose layer, acrylate layer, etc. Reference in this regard is made to U.S. Patents Nos. 4,166,800; 4,389, 330; 4,839,177; and Conte et al., *J. Controlled Release,* Vol. 26. (1993), pages 39-47; all of which are incorporated hereinto by reference in their entirety.

Preferably the active ingredient of the formulation is added in the form of microparticles thereby adding to the time release process.

A first SPH layer or sheet 12, e.g. comprising poly(2-hydroxyethyl methacrylate) ("PHEMA") is formed which is associated e.g. embedded or encapsulated, with the medicament formulation 11 to form an associated region 13. The formulation can be mixed with the SPH monomer prior to polymerization whereby it is encapsulated, physically mixed throughout the matrix or forms part of the matrix or, alternatively, it can be combined with the polymeric matrix of the SPH whereby it is physically imbedded in the surface thereof.

A second SPH layer or sheet 14, e.g. comprising polyacrylic polymers or copolymers, attracted to the first layer 12 is formed which covers at least portion of the region 13. By the term "attracted" is meant that the first layer 12 and the second layer 14 are linked together, either chemically bonded together, e.g. hydrogen bonding, polar bonding, covalent and/or ionic bonding etc. or physically bound together, e.g. by Van Der Waals forces, magnetic forces, etc. This linkage or attraction is accomplished due to substituent groups on their respective surfaces. For example when the layer 12 comprises PHEMA, there are hydroxyl groups (-OH) in its outer or exposed surfaces which hydrogen bond with the exterior or exposed surface carboxyl groups (-COOH) of layer 14 when it comprises polyacrylic acid polymers or copolymers. Accordingly, the hydrogen bonding is a very strong one, typically 10% as strong as a covalent bond, which allows a fairly strong connection or linkage of layers 12 and 14 merely by wetting the surface of each and pressing them together.

It is important to note that the SPH of layers 12 and 14 must be compatible in terms of swelling kinetics within the stomach environment of the patent being treated. If one SPH swells faster than the other a strain will result in the swollen gel which results when the construct or platform is introduced into the stomach, thereby destroying the construct 10 within the stomach. Changing the monomer density and the cross linker density of the SPH will alter the strength and swelling kinetics of the SPH. It is also important to know that the selected hydrogels must have an adequate dwell time or gastric retention in the stomach. In other words the construct or dosage form 10 must remain in the stomach of the patient, e.g. human being, for a desired period of time, e.g. typically from about 1 to about 24 hours.

The construct 10 can be prepared by any conventional means known in the polymer and/or pharmaceutical art, e.g. compression, tabletting technology, spraying technology, encapsulation technology, etc. Preferably, layers or sheets 12 and 14, having the active ingredient associated with layer 12 or 14 or both, are pressed together to encapsulate a deposit of the formulation 11. Typically, sheets or layers 12 and 14 are placed on separate pressure rollers and are fed continuously between the rollers and pressed together. The sheets 12 and 14 are sprayed with the formulation 11 and a conventional wetting agent, e.g. water, voranol, etc., prior to being pressed in this manner.

As previously indicated, the SPHs can swell to hundreds of times their original size in a minute within the stomach. And, of course, the SPH, e.g. in the form of a sheet or film, can be made reasonably strong. If the construct 10 is ingested by a patient, e.g. a human being, and enters the stomach, it swells in the gastric fluid as though placed in water. The construct 10 can be easily ingested if contained in a capsule or other form which will easily degrade, such as in an acidic ambient, like that found in the stomach of an animal, such as a human being, since the construct or platform 10 will not expand in the throat of such animal.

Upon reaching the stomach, the pH sensitive capsule degrades, releasing the inner SPH platform or construct 10. This platform will swell in the stomach, and because of its large size it will be unable to fit through the pyloric sphincter. Housekeeper waves will eventually come into play in order to remove the indigestible platform 10 and so the platform 10 must be strong enough to resist the pressure it will be exposed to.

Inside the SPH platform 10 will be the time release drug formulation 11 that will gradually allow drug to escape into the gastric fluid and become part of the chyme released through the pyloric sphincter. The SPH construct 10 will not hinder this in any way because of the open channels it contains throughout its structure. Essentially, the only purpose of the SPH platform will be to retain the time-release formulation in the stomach.

In an alternative embodiment of the subject invention, a multi-layer or tiered sandwich system or construct is employed. Referring to FIG. 2, there is shown a SPH platform or construct 20 having at least three layers comprising SPH material. The construct 20 has a first layer or sheet 21 comprising a first SPH, e.g. PHEMA, an inner layer 22, comprising a second, different but compatible (in terms of the swelling kinetics) SPH, e.g. polyacrylic acid, etc.; and a third sheet 23 comprising the first SPH or a third, different SPH, which is compatible with the other SPHs employed in layers 21 and 22. The first and third sheets 21 and 23, again, have a functional group or groups, e.g. hydroxy, on their surfaces, which are attracted to a functional group, e.g. carboxyl, on the surface of the inner or second layer or tier 22. Thus, there is provided a linkage between the layers 21 and 23, respectively, and layer 22.

It is to be stressed that a controlled release pharmaceutical formulation can be associated with any single layer 21 or 23, e.g. along their interior surfaces, e.g. mixed, embedded, encapsulated, etc., or both layers 21, 23, or with the inner layer 22, e.g. on its surfaces, alone or with either layer 21, 23 or both layers 21 and 23.

As illustrated in FIG. 2, the inner layer 22 is fabricated whereby a hole, aperture or pocket 24 is formed therein. Aperture 24 is destined to contain the active medicament ingredient or controlled release formulation of a selected drug or medicament (not shown).

As previously discussed, the outer layers or sheets 21, 23 of the platform 20 are treated, e.g. rolled, compressed, etc., whereby they are attracted or linked to the inner sheet 22 and are thus attached thereto whereby the active medicament ingredient or formulation (not shown) contained in the pocket 24 is sealed therein, awaiting introduction into the stomach of the patient being treated.

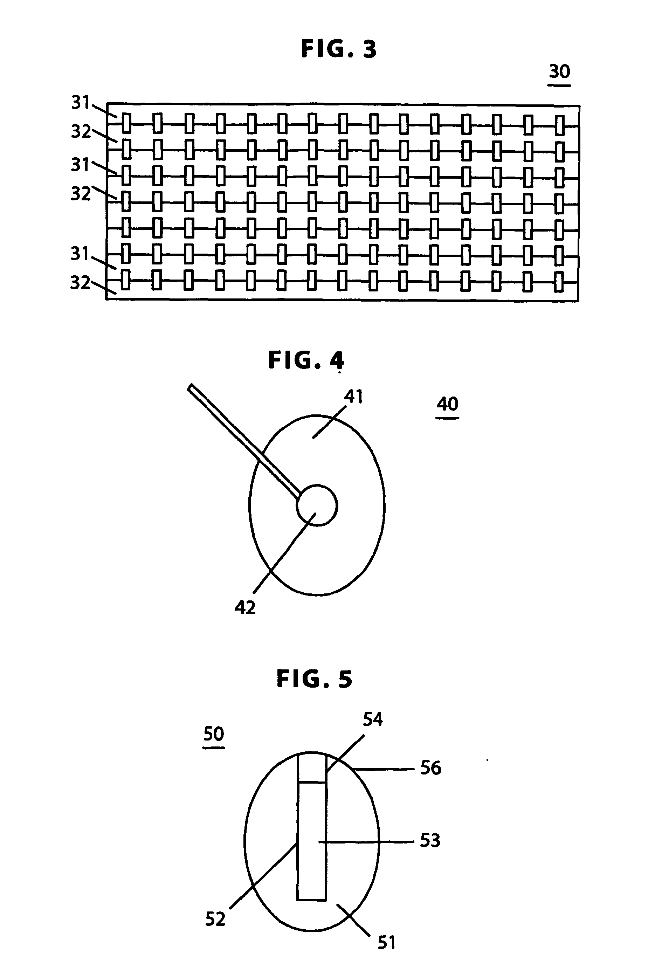

An alternative multi-tiered platform 30 is shown in FIG. 3. The construct or platform 30, has alternating layers or sheets 31 which comprise the same or different but compatible SPHs having surface functional groups which are attracted to the surfaces of the second series of sheets 32 having different but compatible SPHs, as previously described. Applied to or contained within one or both series of sheets 31, 32 is an active ingredient or sustained release formulation (not shown). Typically drug or formulation microparticles are sprayed or deposited in a conventional manner onto the desired surfaces whereby the sprayed or deposited layers are then sealed by the alternating series of sheets whose surfaces are attracted to the deposited surfaces, as previously described.

A core-type platform or construct 40 is described in FIG. 4. A surrounding shell 41 comprising a selected SPH, e.g. P(AA-SPAK), P(HEMA-AA-AM), etc., is formed by any conventional polymerization technique, e.g. free radical polymerization etc., followed by drying. The selected medicament active ingredient or controlled release formulation, e.g. semisolids, microparticulates, etc., is combined with a suitable carrier. A suitable carrier is one which is solid at room temperature, e.g., about 25°C, but which is easily liquified at temperatures slightly above room temperature, e.g. 30 to 85°C, and is inert with the selected medicament. Such a carrier includes long chain fatty acid, wax, polysaturated and glycerides. The medicament is combined with the carrier in its liquid form to form an injectable medicament mixture. The injectable mixture is then injected into the SPH shell 41 using conventional techniques, and the mixture traverses through the pores (not shown) of the shell 41 toward its center to form a solid medicament core 42 upon cooling of the core-type platform 40. The solid core 42 thus remains until the platform 40 enters the stomach of the patient being treated.

A capsule platform system is described in FIG. 5. The capsule 50 comprises a SPH shell 51 which has been polymerized and dried as previously discussed. Contained within an aperture or pocket 52 of the shell 51 is the selected medicament or controlled release formulation 53. At one end 54 of the shell 51 is a plug 56 comprising the same SPH as the shell 51 or a different SPH which is compatible (as to swelling kinetics in the stomach of the patient being treated with the dosage form). The plug 56 assures that the formulation 53 does not escape from pocket 52 during storage and administration to a patient. Any unused space within the pocket 52 is filled with a SPH grain, e.g. P(SPAK), P(AA), P(HEMA).

Typically, the core construct or platform 50 is fabricated in the following manner. The solid dried SPH shell 51 is simply drilled and then sealed with plug 56. The plug 56 may be made to cap the hole or simply fill the end 54. It will be held strongly in place by a slightly faster rate of swelling than the rest of the capsule, or by hydrogen bonding (which is accomplished by using the same method described above). Equally though, the cap 56 may use both properties (hydrogen bonding and faster swelling), or neither. It may be the same superporous hydrogel polymer as the rest of the capsule 51. However, fast swelling of the cap or plug 56 may cause the cap to pop out. Simply drilling a whole block of polymer and cutting cuboid-shaped capsules from the block will produce many units.

The purpose of filling the reservoir with both the active ingredient and ground up superporous hydrogel polymer grain is to ensure that there is no empty or air-filled space in the capsule 50 which could result in the capsule 50 being more easily damaged in the stomach. The ground up polymer grain will swell in the same way as the surrounding walls of the capsule 50, but will allow integration of the active ingredients into the superporous hydrogel platform. The ground-up particles will also act as a kind of plug when they are allowed to swell in the stomach.

Alternatively, the capsule 50, can be fabricated whereby there is a relatively large pocket or cavity 52 and a relatively small entry port or aperture which has to be plugged. The pocket 52 contains not only the formulation 53 but also the plug 56 which comprises a SPH which swells faster than the SPH of the shell 51. Upon introduction into the stomach, the plug 56 swells more rapidly than the remainder of the capsule 50 thereby sealing the pocket 52.

In the construct or platform or dosage form of the invention, the amount of the active ingredient or medicament, depends upon the medicament itself, the rate of administration desired and the metabolism of the patient to be treated. The weight ratio of the first SPH to any other SPHs, e.g. second compatible but different SPH, is typically about 1 to about 1.

It is to be understood that the above-described embodiments are simply illustrative of the principles of the invention. Various other modifications and changes may be devised by those skilled in the art which will embody the principles of the invention and fall within the spirit and scope thereof.

## Claims

1. A pharmaceutical construct which comprises:
(a) at least a first region of the construct comprising a first superporous hydrogel having a medicament associated therewith to form an associated first region;
(b) at least a second region of the construct comprising a second superporous hydrogel, compatible with said first hydrogel, overlying at least a portion of said associated first region and sealing said associated medicament.

2. A medicament platform which comprises:
(a) a first layer comprising a first superporous hydrogel having a first functional group on a surface thereof;
(b) a second layer comprising a second superporous hydrogel having a second functional group on a surface thereof, where said second functional group is linked to said first functional group; whereby said second layer sealably covers at least a portion of said first layer;
(c) a medicament associated with said first layer or said second layer or both said layers and is sealably contained in said portion.

3. The construct as defined in claim 1 wherein said first and said second hydrogels have functional groups on a respective surface which mate and link said respective regions together to form said portion of said associated first region.

4. The construct as defined in claim 1 wherein said first hydrogel is selected from the group consisting of Poly(acrylate acid), Poly(sulfopropyl acrylate), Poly(Hydroxyethyl methacrylate), Poly(acrylic acid-acrylamide) and any mixture of the foregoing hydrogels.

5. The construct as defined in claim 1 wherein said second hydrogel is selected from the group consisting of Poly(acrylate acid), Poly(sulfopropyl acrylate), Poly(hydroxyethyl methacrylate), Poly(acrylic acid-acrylamide) and a mixture of any of the foregoing hydrogels.

6. The platform as defined in claim 2 wherein said first hydrogel is selected from the group consisting of Poly(acrylate acid), Poly(sulfopropyl acrylate), Poly(hydroxyethyl methacrylate), Poly(acrylic acid-acrylamide) and any mixture of the foregoing hydrogels, and said second hydrogel is selected from the group consisting of Poly(acrylate acid), Poly(sulfopropyl acrylate), Poly(hydroxyethyl methacrylate), Poly(acrylic acid-acrylamide) and any mixture of the foregoing hydrogels.

7. The platform as defined in claim 6 wherein said first hydrogel comprises Poly(acrylic acid), and said second hydrogel comprises Poly(hydroxyethyl methacrylate).

8. The platform as defined in claim 2 wherein said medicament is present in an amount ranging from 0.01% to 100% weight percent to the total weight of the platform

9. The platform as defined in claim 2 wherein said first and said second hydrogels comprise a polymer derived from a monomer selected from the group consisting of acrylic acid, sulfopropyl acrylic acid, hydroxyethyl methacrylic acid, acrylic acid - acrylamide and any mixture of the foregoing.

10. An active agent dosage form adapted for gastric retention, comprising:
(a) a first polymeric matrix of a first superporous hydrogel which is swellable when exposed to the stomach environment of an animal being treated;
(b) a second polymeric matrix of a second superporous hydrogel linked to and covering at least a portion of said first polymeric matrix, where said second hydrogel is swellably compatible with said first hydrogel and where each of said first and second matrixes has a surface exposed to the environment of use; and
(c) a therapeutically-effective amount of an active agent associated with at least one of said first and second matrixes defining said portion.

11. The dosage form as defined in claim 10 wherein said first hydrogel is selected from the group consisting of Poly(acrylate acid), Poly(sulfopropyl acrylate), Poly(hydroxyethyl methacrylate), Poly(acrylic acid-acrylamide) and a mixture of any of the foregoing hydrogels.

12. The dosage form as defined in claim 10 wherein said second hydrogel is selected from the group consisting of Poly(acrylate acid), Poly(sulfopropyl acrylate), Poly(hydroxyethyl methacrylate), Poly(acrylic acid-acrylamide) and a mixture of any of the foregoing hydrogels.

13. Use of a construct as defined in claim 1 for the preparation of a medicament for treating a disease condition in a human being or another animal.

14. A method of forming the construct of claim 1 which comprises:
(a) Polymerization to form a polymer;
(b) cleaning and drying the resultant polymer; and
(c) adding said SPH polymer and medicament;
(d) adding to said polymer said SPH polymer and said medicament.

## Patentansprüche

1. Pharmazeutisches Konstrukt, welches umfasst:
(a) mindestens einen ersten Bereich des Konstrukts, umfassend ein erstes superporöses Hydrogel mit einem damit assoziierten Medikament, um einen ersten assoziierten Bereich zu bilden;
(b) mindestens einen zweiten Bereich des Konstrukts, umfassend ein zweites superporöses Hydrogel, das mit dem ersten Hydrogel verträglich ist, und über mindestens einem Abschnitt des ersten assoziierten Bereichs liegt und das assoziierte Medikament abdichtet.

2. Medikamentvorrichtung, welche umfasst:
(a) eine erste Schicht, die ein erstes superporöses Hydrogel mit einer ersten funktionellen Gruppe auf einer Oberfläche derselben umfasst;
(b) eine zweite Schicht, welche ein zweites superporöses Hydrogel mit einer zweiten funktionellen Gruppe auf einer Oberfläche derselben umfasst, wobei die zweite funktionelle Gruppe mit der ersten funktionellen Gruppe verknüpft ist; wobei die zweite Schicht in abdichtbarer Weise mindestens einen Abschnitt der ersten Schicht bedeckt;
(c) ein Medikament, das mit der ersten Schicht oder der zweiten Schicht oder beiden Schichten assoziiert ist, und das in dem Abschnitt in abdichtbarer Weise enthalten ist.

3. Konstrukt wie in Anspruch 1 definiert, wobei die ersten und zweiten Hydrogele funktionelle Gruppen auf ihren jeweiligen Oberflächen aufweisen, welche einander entsprechen und die jeweiligen Bereiche miteinander verknüpfen, um den Abschnitt des ersten assoziierten Bereichs zu bilden.

4. Konstrukt wie in Anspruch 1 definiert, wobei das erste Hydrogel aus der Gruppe ausgewählt wird, die aus Poly(acrylsäure), Poly(acrylsäure-sulfopropylester), Poly(methacrylsäure-hydroxyethylester), Poly(acrylsäure-acrylamid) und einer beliebigen Mischung der vorstehenden Hydrogele besteht.

5. Konstrukt wie in Anspruch 1 definiert, wobei das zweite Hydrogel aus der Gruppe ausgewählt wird, die aus Poly-(acrylsäure), Poly(acrylsäure-sulfopropylester), Poly(methacrylsäure-hydroxyethylester), Poly(acrylsäure-acrylamid) und einer beliebigen Mischung der vorstehenden Hydrogele besteht.

6. Vorrichtung wie in Anspruch 2 definiert, wobei das erste Hydrogel aus der Gruppe ausgewählt wird, die aus Poly-(acrylsäure), Poly(acrylsäure-sulfopropylester), Poly(methacrylsäure-hydroxyethylester), Poly(acrylsäure-acrylamid) und einer beliebigen Mischung der vorstehenden Hydrogele besteht, und wobei das zweite Hydrogel aus der Gruppe ausgewählt wird, die aus Poly(acrylsäure), Poly(acrylsäure-sulfopropylester), Poly(methacrylsäure-hydroxyethylester), Poly(acrylsäure-acrylamid) und einer beliebigen Mischung der vorstehenden Hydrogele besteht.

7. Vorrichtung wie in Anspruch 6 definiert, wobei das erste Hydrogel Polyacrylsäure umfasst, und wobei das zweite Hydrogel Poly(hydroxyethylmethacrylat) umfasst.

8. Vorrichtung wie in Anspruch 2 definiert, wobei das Medikament in einer Menge im Bereich von 0,01 % bis 100 Gew.-%, bezogen auf das Gesamtgewicht der Vorrichtung, vorliegt.

9. Vorrichtung wie in Anspruch 2 definiert, wobei die ersten und zweiten Hydrogele ein Polymer umfassen, das sich von einem Monomer ableitet, welches aus der Gruppe ausgewählt wird, die aus Acrylsäure, Acrylsäure-sulfopropylester, Methacrylsäure-hydroxyethylester, Acrylsäure-Acrylamid und einer beliebigen Mischung der vorstehenden besteht.

10. Dosierungsform für ein wirksames Mittel, die so eingestellt ist, dass sie im Magen zurückgehalten wird, umfassend:
(a) eine erste polymere Matrix eines ersten superporösen Hydrogels, welches quellbar ist, wenn es der Magenumgebung eines zu behandelnden Tieres ausgesetzt wird;
(b) eine zweite polymere Matrix eines zweiten superporösen Hydrogels, das mit der ersten polymeren Matrix verbunden ist und mindestens einen Abschnitt der ersten polymeren Matrix bedeckt, wobei das zweite Hydrogel bezüglich der Quelleigenschaft mit dem ersten Hydrogel verträglich ist und wobei jede der ersten und zweiten Matrizen eine Oberfläche aufweist, die gegenüber der Umgebung für die beabsichtigte Verwendung freiliegt; und (c) eine therapeutisch wirksame Menge eines wirksamen Mittels, das mit mindestens einer der ersten und zweiten Matrizen, welche den Abschnitt definieren, assoziiert ist.

11. Dosierungsform wie in Anspruch 10 definiert, wobei das erste Hydrogel aus der Gruppe ausgewählt wird, die aus Poly(acrylsäure), Poly(acrylsäure-sulfopropylester), Poly-(methacrylsäure-hydroxyethylester), Poly(acrylsäure-acrylamid) und einer beliebigen Mischung der vorstehenden Hydrogele besteht.

12. Dosierungsform wie in Anspruch 10 definiert, wobei das zweite Hydrogel aus der Gruppe ausgewählt wird, die aus Poly(acrylsäure), Poly(acrylsäure-sulfopropylester), Poly-(methacrylsäure-hydroxyethylester), Poly(acrylsäure-acrylamid) und einer beliebigen Mischung der vorstehenden Hydrogele besteht.

13. Verwendung eines Konstrukts wie in Anspruch 1 definiert für die Herstellung eines Medikaments zur Behandlung eines Krankheitszustands in einem Menschen oder einem anderen Tier.

14. Verfahren zur Bildung eines Konstrukts wie in Anspruch 1 definiert, welches umfasst:
(a) Polymerisation, um ein Polymer zu bilden;
(b) Reinigen und Trocknen des erhaltenen Polymers; und
(c) Zugeben des SPH-Polymers und des Medikaments;
(d) Zugeben des SPH-Polymers und des Medikaments zum Polymer.

## Revendications

1. Construction pharmaceutique qui comprend :
(a) au moins une première région de la construction comprenant un premier hydrogel superporeux ayant un médicament associé à celui-ci pour former une première région associée ;
(b) au moins une seconde région de la construction comprenant un second hydrogel superporeux, compatible avec ledit premier hydrogel, recouvrant au moins une partie de ladite première région associée et scellant ledit médicament associé.

2. Plateforme médicamenteuse qui comprend :
(a) une première couche comprenant un premier hydrogel superporeux ayant un premier groupe fonctionnel sur une surface de celle-ci ;
(b) une seconde couche comprenant un second hydrogel superporeux ayant un second groupe fonctionnel sur une surface de celle-ci, où ledit second groupe fonctionnel est lié au dit premier groupe fonctionnel ; de sorte que ladite seconde couche recouvre en la scellant au moins une partie de ladite première couche ;
(c) un médicament associé à ladite première couche ou à ladite seconde couche ou aux deux dites couches et est contenu en la scellant dans ladite partie.

3. Construction telle que définie dans la revendication 1, dans laquelle lesdits premier et second hydrogels ont des groupes fonctionnels sur une surface respective qui correspondent aux dites et lient lesdites régions respectives ensemble pour former ladite partie de ladite première région associée.

4. Construction telle que définie dans la revendication 1, dans laquelle ledit premier hydrogel est choisi dans le groupe constitué de : acide de polyacrylate, polyacrylate de sulfopropyle, polyméthacrylate d'hydroxyéthyle, acide polyacrylique - polyacrylamide et un mélange quelconque des hydrogels susdits.

5. Construction telle que définie dans la revendication 1, dans laquelle ledit second hydrogel est choisi dans le groupe constitué de : acide de polyacrylate, polyacrylate de sulfopropyle, polyméthacrylate d'hydroxyéthyle, acide polyacrylique - polyacrylamide et un mélange de certains quelconques des hydrogels susdits.

6. Plateforme telle que définie dans la revendication 2, dans laquelle ledit premier hydrogel est choisi dans le groupe constitué de : acide de polyacrylate, polyacrylate de sulfopropyle, polyméthacrylate d'hydroxyéthyle, acide polyacrylique - polyacrylamide et un mélange quelconque des hydrogels susdits, et ledit second hydrogel est choisi dans le groupe constitué de : acide de polyacrylate, polyacrylate de sulfopropyle, polyméthacrylate d'hydroxyéthyle, acide polyacrylique - polyacrylamide et un mélange quelconque des hydrogels susdits.

7. Plateforme telle que définie dans la revendication 6, dans laquelle ledit premier hydrogel comprend de l'acide polyacrylique, et ledit second hydrogel comprend du polyméthacrylate d'hydroxyéthyle.

8. Plateforme telle que définie dans la revendication 2, dans laquelle ledit médicament est présent dans une quantité allant de 0,01 % à 100 % en poids par rapport au poids total de la plateforme.

9. Plateforme telle que définie dans la revendication 2, dans laquelle lesdits premier et second hydrogels comprennent un polymère dérivé à partir d'un monomère choisi dans le groupe constitué de : acide acrylique, acide sulfopropylacrylique, acide hydroxyéthylméthacrylique, acide acrylique - acrylamide et un mélange quelconque des susdits éléments.

10. Forme posologique d'agent actif adaptée à la rétention gastrique, comprenant :
(a) une première matrice polymérique d'un premier hydrogel superporeux qui est gonflable lorsqu'elle est exposée à l'environnement de l'estomac d'un animal qui est traité ;
(b) une seconde matrice polymérique d'un second hydrogel superporeux liée à et recouvrant au moins une partie de ladite première matrice polymérique, où ledit second hydrogel est compatible par gonflement avec ledit premier hydrogel et où chacune desdites première et seconde matrices comprend une surface exposée à l'environnement d'utilisation ; et
(c) une quantité thérapeutiquement efficace d'un agent actif associé à au moins l'une desdites première et seconde matrices définissant ladite partie.

11. Forme posologique telle que définie dans la revendication 10, dans laquelle ledit premier hydrogel est choisi dans le groupe constitué de : acide de polyacrylate, polyacrylate de sulfopropyle, polyméthacrylate d'hydroxyéthyle, acide polyacrylique - polyacrylamide et un mélange de certains quelconques des hydrogels susdits.

12. Forme posologique telle que définie dans la revendication 10, dans laquelle ledit second hydrogel est choisi dans le groupe constitué de : acide de polyacrylate, polyacrylate de sulfopropyle, polyméthacrylate d'hydroxyéthyle, acide polyacrylique - polyacrylamide et un mélange de certains quelconques des hydrogels susdits.

13. Utilisation d'une construction telle que définie dans la revendication 1, pour la préparation d'un médicament pour traiter un état pathologique chez l'homme ou un autre animal.

14. Procédé de formation de la construction selon la revendication 1, qui comprend :
(a) la polymérisation pour former un polymère ;
(b) le nettoyage et le séchage du polymère résultant ; et
(c) l'addition dudit polymère SPH et du médicament ;
(d) l'addition au dit polymère dudit polymère SPH et dudit médicament.
